# EUROPÄISCHE PATENTSCHRIFT

(11) **EP 2 422 288 B1**
(45) Veröffentlichungstag und Bekanntmachung des Hinweises auf die Patenterteilung: **16.10.2019**
(21) Anmeldenummer: 10715715.8
(22) Anmeldetag: 22.04.2010
(51) Int. Cl.: G16H 40/63

(54) **MEDIZINISCHE BEHANDLUNGSVORRICHTUNG**
MEDICAL TREATMENT APPARATUS
DISPOSITIF DE TRAITEMENT MÉDICAL

(30) Priorität: 24.04.2009 DE 102009018806
(43) Veröffentlichungstag der Anmeldung: 29.02.2012
(73) Patentinhaber: Fresenius Medical Care Deutschland GmbH, 61352 Bad Homburg (DE)
(72) Erfinder: BRÜGGERHOFF, Arnd, 65929 Frankfurt (DE); GRÜNDKEN, Martin, 61191 Rosbach (DE); RIPKEN, Andreas, 61267 Neu-Anspach (DE)
(74) Vertreter: Bobbert & Partner Patentanwälte PartmbB
(86) Internationale Anmeldenummer: PCT/EP2010/002492
(87) Internationale Veröffentlichungsnummer: WO 2010/121820

(56) Entgegenhaltungen:
- EP-A1- 0 608 762
- US-A- 5 230 061
- US-A1- 2002 101 451
- US-A1- 2007 215 545

## Beschreibung

Die vorliegende Erfindung betrifft eine medizinische Behandlungsvorrichtung gemäß dem Oberbegriff des Anspruchs 1.

Medizinische Behandlungsvorrichtungen, wie beispielsweise Dialysevorrichtungen, werden mittels einer i. a. R. umfangreichen Gerätesoftware gesteuert oder geregelt. Diese Software umfasst oftmals mehrere tausend Programmzeilen, welche regelmäßig in höheren und wenig intuitiv zugänglichen Programmsprachen wie C⁺⁺ programmiert sind. Der einer solchen Software zugrunde liegende Quellcode wird i. a. R. mit jedem Software-Update und mit jeder neuen Softwareversion umfangreicher und oftmals auch komplexer. Änderungen der Software, wie sie beispielsweise für ein Update erforderlich sind, müssen daher von einem hochqualifizierten Programmierer eingepflegt werden. Da das Erstellen von Software noch immer eine in höchstem Maße individuelle Arbeit darstellt, müssen Änderungen oftmals sogar von demselben Programmierer durchgeführt werden, welcher bereits auch die vorhergehenden Softwareversionen programmiert hat, möchte man zusätzlichen Aufwand und Kosten für das Einarbeiten eines weiteren Programmierers in den bereits vorliegenden Programmaufbau und -stil vermeiden. Der Aufwand beim Umsetzen erforderlicher Änderungen an der Software ist jedoch in jedem Fall außerordentlich hoch. Dasselbe gilt für die hiermit verbundenen Kosten.

Änderungen an der Software aufgrund von gewandelten Anforderungen an das Ablaufverhalten der mit der Software betriebenen medizinischen Behandlungsvorrichtung erfordern nach jeder Änderung ein erneutes Qualifizieren nicht nur der geänderten, sondern auch aller bisherigen Funktionen des gesamten Geräts zum Überprüfen dessen einwandfreier Funktion. Dies kann bei komplexeren Systemen, wie beispielsweise Dialysevorrichtungen, zu einer oftmals unüberschaubar großen Anzahl von zu testenden Fällen und entsprechendem Aufwand führen.

Da zum Ändern des Ablaufverhaltens durch Eingriff in die Software bislang Programmierkenntnisse erforderlich sind, können allein Softwareentwickler das Ablaufverhalten der Gerätesoftware verändern. Verfahrensentwickler, welche i. a. R. keine Softwarespezialisten sind, können diese Änderungen hingegen oftmals nicht durchführen, obwohl sie wiederum die medizinischen Behandlungsverfahren am besten kennen und zum Modifizieren oder Ändern des Ablaufverhaltens der entsprechenden Vorrichtungen besonders gut ausgebildet sind. Auch diese Tatsache führt zu Aufwand und Kosten aufgrund erforderlicher Abstimmung zwischen Programmierer und Verfahrensentwickler. Zudem steigt die Fehlerwahrscheinlichkeit der Arbeit aufgrund von Kommunikationsschwierigkeiten an.

Aus der US 2002/0101451 A1 ist ein Programmiersystem einer speicherprogrammierbaren Steuerung bekannt.

Der vorliegenden Erfindung liegt die Aufgabe zugrunde, eine medizinische Behandlungsvorrichtung anzugeben.

Die erfindungsgemäße Aufgabe wird gelöst durch die Merkmalskombination des Anspruchs 1.

Bei der Datenbank des Anspruchs 1 kann es sich um eine dem Fachmann bekannte SQL-Datenbank oder jede andere geeignete Datenbank handeln.

Im Speicher oder in der Datenbank können Daten, die für den Betrieb einer medizinischen Behandlungsvorrichtung relevant sind, flüchtig (nur während bestehender Versorgung von Speicher oder Datenbank mit Strom oder Energie) oder dauerhaft (auch noch nach Trennung von einer Strom- oder Energiezufuhr) gespeichert und gegebenenfalls gewartet werden.

Die gespeicherten Daten können vorzugsweise Teile oder die Gesamtheit aller für den Betrieb der medizinischen Behandlungsvorrichtung relevanten Daten sein oder umfassen.

Die gespeicherten Daten können vorzugsweise zur Parametrisierung von behandlungsspezifischen Abläufen verwendet werden, die in festen Programmabschnitten codiert sind.

Diese Daten können in Form eines oder mehrerer Parametersätze oder eines Quellcodes wenigstens einer ersten Programmiersprache vorliegen. Sie können auch in Form einer aus einem solchen Parametersatz oder Quellcode einer ersten Programmiersprache entstandenen Datei oder in Form eines aus diesem entstandenen Programms - insbesondere eines nicht maschinenlesbaren Programms - vorliegen.

Das System, welches durch einen Einzelrechner oder einen Verbund mehrerer Geräte - Rechner oder anderer Vorrichtungen - dargestellt sein kann, weist ferner wenigstens eine Eingabeeinrichtung auf.

Die Eingabeeinrichtung kann eine Schnittstellensoftware umfassen oder aus dieser bestehen.

Die Eingabeeinrichtung kann für ein manuelles Eingeben von Daten wie eine Tastatur, eine Computermaus, ein Stick oder dgl. ausgestaltet sein. Sie kann jedoch auch auf jede andere, dem Fachmann für den erfindungsgemäßen Zweck geläufige Weise ausgestaltet sein.

Die Eingabeeinrichtung kann zur Erleichterung der Eingabe und zur Vermeidung fehlerhafter Eingaben aufbereitete Formularblätter bereithalten, in welche der Anwender ohne besondere Programmierkenntnisse, und gegebenenfalls vom System geführt, Eingaben vornehmen kann, welche Änderungen oder neue Routinen im Ablaufverhalten der medizinischen Behandlungsvorrichtung widerspiegeln.

Die Schnittstelle oder die Formularblätter können allgemein verständlich aufgebaut sein. Sie können mit Benutzerhilfen versehen sein.

Die Eingaben können dabei vom System auf Logik und Vereinbarkeit mit anderen Eingaben oder bekannten übrigen Daten überprüft werden. Auffälligkeiten hierbei können dem Benutzer mitgeteilt werden.

Eingaben in die Eingabeeinrichtung oder die hierfür vorgesehenen Formularblätter erfordern somit nicht die Mithilfe eines hochqualifizierten Softwareentwicklers, sondern können z. B. vom Verfahrensentwickler selber vorgenommen werden.

Die Datenbank und die Eingabeeinrichtung können sich auf einem Arbeitsrechner des Verfahrenentwicklers befinden. Sie sind somit unabhängig von der Präsenz der medizinischen Behandlungsvorrichtung bedienbar.

Die mittels der Eingabeeinrichtung einzugebenden Daten, welche das Ablaufverhalten der medizinischen Behandlungsvorrichtung betreffen, können dabei in einem Format eingegeben werden, welches dem Format des wenigstens einen Parametersatzes und/oder einer anderen als der ersten Programmiersprache und/oder hieraus entstandenen Dateiformaten oder dergleichen entspricht.

Die eingegebenen Daten können in der Datenbank gespeichert und dort gegebenenfalls gewartet werden.

Das System weist ferner wenigstens eine Ausgabeeinrichtung, beispielsweise in Form eines Monitors, eines Druckers oder dergleichen, zum Erzeugen und Ausgeben einer Ablaufdefinitionsausgabedatei auf. Die Ausgabeeinrichtung ist erfindungsgemäß zum vorgenannten Erzeugen und Ausgeben konfiguriert.

Die Ablaufdefinitionsausgabedatei kann eine Textdatei im Format "*.csv" (character-separated values) sein.

In der Ablaufdefinitionsausgabedatei können alle relevanten und/oder zu ändernden Abläufe und/oder neu einzugebenden Abläufe oder Routinen, vorzugsweise mit allen erforderlichen Einzelheiten, für den Verfahrensentwickler nachvollziehbar, gegebenenfalls entsprechend aufbereitet, aufgeführt sein.

Eine Überprüfung der mittels der Eingabeeinrichtung eingegebenen Daten kann daher beispielsweise zur Vermeidung oder zum einfacheren Auffinden von Eingabefehlern übersichtlich und transparent anhand der Ablaufdefinitionsausgabedatei erfolgen. Sie trägt damit vorteilhaft zur Verringerung der Fehlerhäufigkeit beim Programmieren bei.

Die Ablaufdefinitionsausgabedatei kann dabei am Monitor dargestellt werden, sie kann jedoch auch auf Papier ausgegeben werden. Letzteres kann einer besseren Lesbarkeit dienen und damit die Gefahr, einen Fehler in der Datei beziehungsweise in den zu definierenden Abläufen zu übersehen, vorteilhaft senken. Ferner kann auch jede andere geeignete Wiedergabeform genutzt werden.

Das System weist ferner wenigstens eine Konvertierungseinrichtung - diese kann ein Computer, eine Einrichtung hiervon und/oder ein Computerprogramm sein - zum Erzeugen von wenigstens einer maschinenlesbaren Konfigurationsdatei, basierend auf dem Parametersatz, auf. Die Konvertierungseinrichtung ist erfindungsgemäß zum vorgenannten Erzeugen konfiguriert.

In der Konvertierungseinrichtung kann eine weitere (Schnittstellen-)Software zum Einsatz kommen.

Mittels der Konvertierungseinrichtung wird die Ablaufdefinitionsausgabedatei beispielsweise in sogenannte Header-Dateien ("*.hpp") konvertiert.

Das Format der Dateien, in welche konvertiert wird, kann vorzugsweise in der verwendeten Programmiersprache mittels des Compilers verarbeitet werden. Beispielsweise können dies für C⁺⁺ "*.hpp"-Header-Dateien sein.

Mittels der Konvertierungseinrichtung können - insbesondere bedarfsgerecht adaptierte - maschinenlesbare Dateien - auch Code-Dateien genannt - oder maschinenlesbare Daten erzeugt werden. Die maschinenlesbare Datei oder die maschinenlesbaren Daten in einem Speichermedium (die beiden letztgenannten Begriffe werden in der vorliegenden Anmeldung austauschbar verwendet) können dabei als eine einzige Datei oder auch als eine Gruppe von verbundenen oder zusammenwirkenden Dateien zu verstehen sein, ferner auch als wenigstens eine Datei mit wenigstens einer Unterdatei.

Unter einem "Konvertieren" kann erfindungsgemäß insbesondere eine Formatwandlung der Ablaufdefinitionsdatei oder der hierin enthaltenen Daten in eine maschinenlesbare Form zu verstehen sein. Vorzugsweise werden bei diesem Vorgang keine weiteren, über jene Informationen der Datenbank oder der Ablaufdefinitionsausgabedatei hinausgehenden Informationen hinzugefügt, umgewandelt oder gespeichert. Insbesondere wird vorzugsweise keine für den Betrieb der medizinischen Behandlungsvorrichtung relevante Information hinzugefügt, umgewandelt oder gespeichert.

Das System kann wie oben erwähnt auf einem Rechner des Verfahrensentwicklers ausgeführt werden. Sein Ergebnis (z. B. die generierten Header-Dateien) kann zusammen mit dem Geräte-Betriebsprogramm und einer entsprechenden Ablaufermittlungseinrichtung unter Verwendung eines Compilers in einen Programmcode umgewandelt werden, der z. B. in einem Speicher einer medizinischen Behandlungseinrichtung abgelegt und dort ausgeführt wird.

So wird in einer bevorzugten Ausführungsform vorgeschlagen, dass die Ausgabeeinrichtung geeignet und vorbereitet ist, eine Ablaufdefinitionsausgabedatei mit wenigstens einer Tabelle in Zeilen- und Spaltenform auszugeben.

Eine solche Ausgabeform eignet sich aufgrund ihrer Untergliederung in Spalten, Zeilen, Zellen und Tabellenbereiche zu einer besonders aussagekräftigen und übersichtlichen Darstellung der mittels der Eingabeeinrichtung eingegebenen Ablaufdefinitionen. Diese Darstellung hilft somit der Reduzierung der Gefahr, einen Eingabefehler zu übersehen.

In einer weiter bevorzugten Ausführungsform weist das System eine Ausgabeeinrichtung zur Ausgabe einer Tabelle der Ablaufdefinitionsausgabedatei auf, welche in Blöcke unterteilt ist. Dabei weist die Tabelle wenigstens Informationen aus einer Gruppe auf, welche wenigstens Erkennungszustände, einen Verteilerschlüssel, einen Verfahrensblock, einen Systemzustandsblock und einen Reaktionsblock umfasst. Diese Informationen können in Zeilen und/oder Spalten entsprechend aufbereitet sein. Dabei kann der Reaktionsblock vordefinierte Informationen enthalten, die beispielsweise zu einer alarmbedingten Betätigung von Aktoren der medizinischen Behandlungsvorrichtung, welche mit dem Ergebnis des Systems betrieben wird (Klemmen, Ventile, Pumpenantriebe etc.), führen.

Die Blöcke können jeweils eine festgelegte oder eine dynamisch oder anderweitig veränderbare Anzahl von Spalten und/oder Zeilen belegen. Jede Zelle kann dabei beispielsweise entweder den Eintrag "1" oder "0" tragen, je nachdem ob ein Zustand zutrifft ("1") oder nicht ("0"). Dies vereinfacht die Überprüfung der eingegebenen Werte. Es ist erfindungsgemäß möglich, die Ablaufdefinitionsausgabedatei unter Verwendung eines kommerziellen Tabellenkalkulationssoftwareprogramms, wie z. B. Microsoft-Excel™, auszuführen, in welchem in einem Arbeitsblatt in bekannter Weise Zellen durch Einträge in Zeilen- und Spaltenform belegt werden. Zeilen, die dabei alle Blöcke übergreifen, können jeweils eine der erdenklichen Ablaufsituationen und -reaktionen aufgrund der Kombinationsmöglichkeiten aus Verfahrensauswahl und Systemzustand abbilden.

Dabei kann die Ablaufdefinitionsausgabedatei in einer besonders bevorzugten erfindungsgemäßen Ausführungsform eine Alarmdefinitionstabelle (kurz ADT) umfassen. Die Tabellenform ist eine besonders übersichtliche Darstellungsform, in welcher sich Abläufe und Zusammenhänge wie beispielsweise Alarmzustände auf einfache, intuitiv zu erfassende Weise darstellen lassen.

Der Datenträger kann, wie in einer weiter bevorzugten Ausführungsform vorgeschlagen, eine Mehrzahl von mittels des Systems erzeugten maschinenlesbaren Dateien oder erzeugten maschinenlesbaren Daten aufweisen, wobei sich wenigstens zwei Dateien aus der Mehrzahl von maschinenlesbaren Dateien oder Sätzen von maschinenlesbaren Daten voneinander unterscheiden. Das Vorsehen unterschiedlicher maschinenlesbarer Dateien ist insbesondere dann sinnvoll und vorteilhaft, wenn eine medizinische Behandlungsvorrichtung eine Mehrzahl von CPUs aufweist, von welchen wenigstens zwei CPUs unterschiedliche Informationen beim Betrieb der medizinischen Behandlungsvorrichtung benötigen.

Der Datenträger kann dabei ein üblicher Speicher sein. Dieser kann vorübergehend speichern, er kann aber auch ein Dauerspeicher sein. Es kann sich um einen stationären Speicher oder um einen vornehmlich transportablen Datenspeicher handeln.

Die erfindungsgemäße medizinische Behandlungsvorrichtung kann insbesondere als Dialysevorrichtung, beispielsweise zum Durchführen einer HD (Hämodialyse), HDF (Hämodiafiltration), einer CVVH (Continuous Venous Venous Hemofiltration), CVVHD (Continuous Venous Venous Hemodialysis) CVVHDF (Continuous Venous Venous HemoDiaFiltration) oder anderer Therapieverfahren, ausgestaltet sein.

Vorteilhafte Weiterbildungen der medizinischen Behandlungsvorrichtung sind wiederum Gegenstand der Unteransprüche.

So weist die medizinische Behandlungsvorrichtung in einer bevorzugten Ausführungsform ein ROM auf, in welchem die mittels des Systems erzeugte maschinenlesbare Datei oder ein solche Dateien aufweisender Datenträger abgelegt oder gespeichert ist. In einer weiter bevorzugten Ausführungsform weist die medizinische Behandlungsvorrichtung eine Ablaufermittlungseinrichtung auf. In einer wiederum weiteren erfindungsgemäßen Ausführungsform weist die medizinische Behandlungsvorrichtung ein ROM auf, in welchem eine Alarmzustandsermittlungseinrichtung bzw. ein Alarmhandler-Code abgelegt ist.

Mittels der vorliegenden Erfindung (d. h. mittels der medizinischen Behandlungsvorrichtung) ist es vorteilhaft möglich, Ablaufdefinitionen zentralisiert festzulegen, zu speichern und bei Bedarf auch zu ändern. Die Ablaufdefinitionen, bei welchen es sich beispielsweise um Alarmdefinitionen - also Definitionen von Alarmzuständen - handeln kann, können mittels der vorliegenden Erfindung im Wesentlichen aus dem Quellcode der zum Betreiben der medizinischen Behandlungsvorrichtung verwendeten Software "herausgezogen" werden. Hierzu wird eine wartungsfreundliche, modulare Struktur vorgeschlagen, bei welcher alle zur Darstellung des Systemverhaltens erforderlichen Informationen an einer gemeinsamen Stelle verwaltet und in einer Form dargestellt werden können, welche auch für einen Nichtprogrammierer, z. B. den Verfahrensentwickler, eingebbar und beispielsweise zu Kontrollzwecken zuverlässig lesbar ist. Die Qualifizierung der Gerätesoftware reduziert sich daher auf die Kontrolle der eingegebenen Angaben und Strukturen und gegebenenfalls auf Stichproben des Geräteverhaltens nach vorgenommener Änderung an der Gerätesoftware.

Ein weiterer Vorteil des Systems besteht darin, dass sich nach erfolgter Erstqualifizierung von eingegebenen Änderungen oder Ergänzungen der Aufwand für weitere Änderungen erheblich reduziert, verglichen mit den aus dem Stand der Technik bekannten Verfahren oder Systemen. Dort erhöht sich der Qualifizierungsaufwand mit jeder weiteren Änderung noch zusätzlich erheblich.

Ein weiterer Vorteil des Systems und des diesem zugrunde liegenden Verfahrens besteht in der transparenten und einfachen Programmierung. Dies führt dazu, dass Fehler in Abläufen und im Quellcode wirksam reduziert oder gar verhindert werden.

Der erfindungsgemäß vorgeschlagene Ansatz erlaubt es damit vorteilhaft, alle zur Darstellung des Systemverhaltens notwendigen Informationen an einer zentralen Stelle zu verwalten und in einer Form darzustellen, die auch für einen Nicht-Softwareentwickler "lesbar" ist.

Somit kann erfindungsgemäß mit geringem Aufwand und nahezu ohne Programmierkenntnisse eine "Umprogrammierung" oder ein Update der Gerätesoftware oder von Teilen hiervon einer medizinischen Vorrichtung, wie z. B. einer Dialysevorrichtung, zuverlässig erfolgen.

Die vorliegende Erfindung wird im Folgenden anhand der beigefügten Zeichnung exemplarisch im Detail erläutert. In der Figurenbeschreibung werden Begriffe verwendet, die im Folgenden mit Geltung allein für die Figurenbeschreibung definiert werden. Dabei gilt:
"ACG" steht für "Advanced Code Generator", ein Hilfsprogramm, das die Daten der ADT (im CSV-Format) in ein internes Format umsetzt, welche anschließend vom Compiler bzw. von der Konvertierungseinrichtung gelesen werden kann.

"ADT" steht für "Alarm Definition Table". Dies ist wenigstens eine Datei im CSV-Format, in der alle für die Alarmverarbeitung erforderlichen Informationen enthalten sind. Die ADT ist aufgeteilt in die Vorverarbeitungstabelle, die Erkennungstabelle und die Relevanztabelle.

"Alarmhandler" oder Alarmhandler-Code steht für einen Teil der Gerätesoftware; der Alarmhandler stellt einen Codeteil dar, der die aus der ADT entnommenen (und mit dem ACG umgeformten) Informationen bearbeitet und die in der ADT definierten Reaktionen auslöst.

"AWI" steht für ein Präfix, das sich auf das Meldungshandling bezieht (Alarm, Warnung, Info).

"AWI-ID" steht für eine eindeutige Nummer zur Zuordnung und Bezeichnung einer Meldung.

Unter einem Behandlungsverfahren wird die Ablaufdefinition für eine medizinische Behandlung verstanden, beispielsweise CVVHDF, SCUF, HD-Verfahren und HDF-Verfahren. In einer Gerätesoftware ist i. d. R. die Möglichkeit zur Durchführung verschiedener Behandlungsverfahren implementiert. Das aktuell durchzuführende Behandlungsverfahren wird vom Anwender festgelegt.

"Compiler" steht für ein kommerzielles Programm, das aus dem Quellcode (geschrieben in einer Programmiersprache) einen Binärcode (Programmcode) erstellt, der vom jeweiligen Mikroprozessor verarbeitet wird.

"CPU" ist ein Synonym für den Mikroprozessor; ein Gerät bzw. eine Vorrichtung kann mehrere CPUs enthalten, die sich eine Gesamtaufgabe teilen.

"CSV-Format" steht für "Character-Separated Values", ein im EDV-Bereich übliches textbasiertes Austauschformat, das sich beispielsweise mit Microsoft Excel™ strukturiert visualisieren lässt.

Die "Enable-Spalte" ist Teil der Erkennungstabelle; in der Enable-Spalte wird festgelegt, ob die Bedingungsdefinition in dieser Zeile beginnt, oder ob eine UND-Verknüpfung mit einer vorherigen Tabellenzeile besteht.

In der "Erkennungstabelle" werden Werte (aus Variablen und/oder Konstanten) mit Bedingungen verknüpft. Das Ergebnis einer Verknüpfung ist ein "Erkennungszustand".

Ein "Erkennungszustand" steht für ein Ergebnis einer Vergleichsverknüpfung. Er stellt einen logischen Zustand (wahr oder falsch) dar, je nachdem, ob die zugrundeliegende Bedingung erfüllt ist oder nicht.

Die "Gerätesoftware" umfasst alle Module (Daten- und Programmmodule), die vom Compiler in den Programmcode umgesetzt werden. Wenn im Gerät mehrere Mikroprozessoren eingesetzt werden, umfasst die Gerätesoftware alle Module aller Prozessoren.

Ein "Gerätezustand" ist der Zustand einer oder mehrerer Gerätekomponenten, wie z. B. "Klemme 1 ist auf", "Druck A ist größer als <Wert>".

"Internes Format" bedeutet am Beispiel des C/ C⁺⁺ - Compilers die Anordnung von Daten in Header-Dateien (z. B. "*.H" oder "*.HPP").

Der "Programmcode" (auch Binärcode) liegt im Speicher des Gerätes vor und wird im Betrieb des Gerätes von den entsprechenden Prozessoren abgearbeitet.

Ein "Systemzustand" signalisiert, dass sich innerhalb eines Behandlungsablaufes das Gerät in unterschiedlichen Zuständen wie "Initialtest", "Gerätetest", "Aufrüsten", "Vorbereiten", "Füllen", "Spülen", "Behandeln", "Fehler", "Ausschalten", etc. befinden kann.

"Sensorwerte" sind Variable, die Werte zugehöriger Sensoren angeben. Dies sind z. B. Drücke, Spannungen, Flusswerte, etc.

Unter "Subverfahren" werden Alternativen oder optionale Zusätze innerhalb eines Behandlungsverfahrens verstanden. So kann z. B. die Blutgerinnungshemmung innerhalb jedes Verfahrens durch Heparinisierung oder Citrat/Calcium-Gabe als Beispiel eines Subverfahrens unterstützt werden.

Die "Systemreaktion" definiert das Verhalten des Gerätes in einem bestimmten Gerätezustand. Die Systemreaktion ist komponentenbasiert (Pumpen, Klemmen, usw.). Die Gesamtheit aller Systemreaktionen ergibt im Wesentlichen das Systemverhalten.

Das "Systemverhalten" bestimmt, wie das Gerät mit Werten und Zuständen umgehen soll. Das Systemverhalten wird durch die Programmierung (Gerätesoftware) realisiert.

"Verfahrenszustand" ist ein Sonderzustand des Gerätes, der sich auf mehrere Komponenten bezieht; z. B. können definiert sein:

| | |
|---|---|
| SZ_Blut | Blutsystem angehalten |
| SZ_Bilanz | Bilanzierung angehalten |
| SZ_FPSA | FPSA angehalten |
| SZ_CiCa | CiCa angehalten |
| SZ_Heparin | Heparin angehalten |
| SZ_PatDisconn | Patient diskonnektiert. |

Der Begriff "Vorverarbeitungstabelle" steht für einen Teilbereich der ADT und definiert, ob und wie einzelne Sensor- und Zustandswerte vorverarbeitet werden.

Vorverarbeitungen sind z. B. arithmetische Mittelungen, Ermittlungs- und Eliminierungsalgorithmen für Artefakte, etc.

In der Zeichnung bezeichnen gleiche Bezugszeichen gleiche oder identische Strukturen. Es gilt:
- Fig. 1: zeigt einen schematischen Aufbau eines Systems in seiner Zusammenwirkung mit einer erfindungsgemäßen medizinischen Behandlungsvorrichtung;
- Fig. 2: zeigt ein Beispiel einer Relevanzbewertung, um bei einem positiven Ergebnis eine entsprechend vordefinierte Reaktion der medizinischen Behandlungsvorrichtung auszulösen;
- Fig. 3: zeigt in Form eines Schaubildes Daten- und Programmstrukturen in ihrem Bearbeitungsablauf;
- Fig. 4: gibt eine erfindungsgemäße Tabelle mit Parametern für eine Vorverarbeitung wieder;
- Fig. 5a und 5b: geben eine Erkennungstabelle wieder; und
- Fig. 6a bis 6f: geben eine Relevanztabelle wieder.

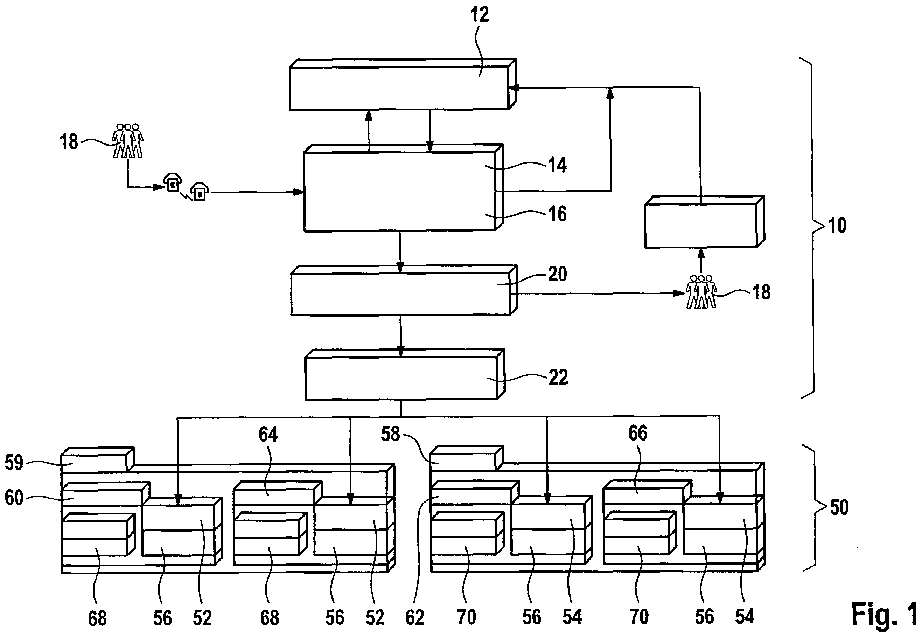

Fig. 1 zeigt ein System 10 sowie Abschnitte einer erfindungsgemäßen medizinischen Behandlungsvorrichtung 50, welche im Beispiel der Fig. 1 als Dialysevorrichtung ausgestaltet ist. Dabei ist das System 10 außerhalb der medizinischen Behandlungsvorrichtung 50 und unabhängig von dieser angeordnet. Das System 10, welches beispielsweise auf dem externen Rechner eines Verfahrensentwicklers vorliegt, weist u. a. eine SQL-Datenbank 12 zum Speichern und Verwalten von für den Betrieb der medizinischen Behandlungsvorrichtung 50 relevanten Daten auf.

Eingaben und Ausgaben von Dateien in das System 10 und insbesondere in die Datenbank 12 erfolgen über ein eigenes Tool, nämlich die Eingabeeinrichtung 14, welche im Beispiel der Fig. 1 zugleich als Ausgabeeinrichtung 16 verwendet wird. Dieses Tool kann in dem Fall, dass das System zur Festlegung von Alarmen dient, als Alarm Database Interface (ADI) bezeichnet werden, wie dies im Folgenden auch der Fall ist. Im Tool 14, 16 werden Formularblätter für die Eingabe verwendet, welche sowohl von Softwareentwicklern als auch von Verfahrensentwicklern genutzt werden. Die Eingabe mittels Formularblättern oder -masken erlaubt es vorteilhaft, dass bei gewünschten Änderungen der Reaktion der Geräte - beispielsweise im Alarmfall, in welchem die Maschine nicht mehr nur akustisch, sondern zusätzlich auch optisch einen Alarm anzeigen soll - nicht mehr der Softwareingenieur einen neuen Quellcode formulieren muss, sondern Verfahrensentwickler 18 die entsprechenden Eingaben an einem separaten Arbeitsplatz über ein Eingabeformular der Eingabeeinrichtung 14 vornehmen und damit ein neues Alarmverhalten der Maschine definieren oder vorgeben können.

Sind alle Erkennungszustände bzw. alle für die Änderung erforderlichen Daten eingegeben, so erzeugt die Ausgabeeinrichtung bzw. das ADI-Tool 16 eine Ablaufdefinitionsausgabedatei, welche im Beispiel der Fig. 1 eine Textdatei (*.csv) in Form einer Tabelle, der sogenannten "Alarm Definition Table" oder ADT 20, ist. In dieser Ablaufdefinitionsausgabedatei bzw. ADT 20 sind alle relevanten oder gar erdenklichen Alarmsituationen und -reaktionen tabellarisch aufgelistet. In dieser Form sind die eingegebenen Daten für das zuvor erwähnte oder ein anderes Entwicklerteam 18 leicht zu überprüfen. Dies kann beispielsweise mittels Papier-Review erfolgen. Sollten Änderungen in der Ablaufdefinitionsausgabedatei 20 erforderlich sein, so werden diese wiederum über Eingabeformulare der Eingabeeinrichtung 14 in der SQL-Datenbank 12 abgelegt, vereinfacht und erneut als Textdatei tabellarisch ausgegeben.

Es können dabei beliebig viele Review-Schritte durchgeführt werden. Da jedoch immer nur die Änderungen zur vorherigen Version überprüft werden müssen, können zusätzliche Ablaufdefinitionen oder Alarmdefinitionen und Anweisungen für ein entsprechend geändertes Alarmverhalten ohne großen Aufwand hinzugefügt werden.

Ist das Ablauf- oder Alarmverhalten abschließend in der Textdatei beschrieben bzw. hinterlegt und qualifiziert bzw. überprüft, so wird die Ablaufdefinitionsausgabedatei bzw. ADT 20 von einer Konvertierungseinrichtung 22, welche im Beispiel der Fig. 1 als ein so genannter Alarm Code Generator (kurz: ACG) ausgestaltet ist, in eine maschinenlesbare Datei oder in einem Speichermedium vorliegende Daten 52, 54, die in Fig. 1 als ein sogenanntes Headerfile (*.hpp) ausgeführt ist, konvertiert.

In dem in Fig. 1 dargestellten Abschnitt der medizinischen Behandlungsvorrichtung 50 ist eine Ablaufermittlungseinrichtung in Form eines Alarmhandler-Codes 56 vorgesehen, mittels welcher die maschinenlesbaren Dateien 52, 54 - bei welchen es sich ganz allgemein auch um gespeicherte maschinenlesbare Daten handeln kann - auslesbar sind.

Dabei können mittels der Konvertierungseinrichtung 22 die verschiedenartigen CPUs der medizinischen Behandlungsvorrichtung berücksichtigt werden. D. h., es wird stets ein maschinenlesbarer Code identischer Struktur erzeugt, jedoch wird er individuell auf die verschiedenen Funktionen und Anforderungen der unterschiedlichen CPUs der medizinischen Behandlungsvorrichtung 50 angepasst. So muss beispielsweise die Operations-CPU 62 des Monitors 58 nicht exakt jene Information erhalten, welche die Schutz-CPU 64 der Verfahrenssteuerung 59 z. B. zum Schließen einer venösen Klemme erhält. Die konvertierten oder kompilierten Codes, welche in den maschinenlesbaren Dateien 52, 54 vorliegen, können während der Produktion der medizinischen Behandlungsvorrichtung 50 beispielsweise in den ROM-Speicher der ausgelieferten Behandlungsvorrichtung 50 eingebrannt werden und sind damit unveränderlich. Die maschinenlesbaren Dateien 52, 54 können jedoch auch nach Auslieferung der medizinischen Behandlungsvorrichtung 50 an den Benutzer im Rahmen eines Updates zu einem späteren Zeitpunkt in die Behandlungsvorrichtung 50 integriert werden. Die Vorschriften zur Verarbeitung der Ablaufdefinition sind auf jeder CPU identisch im Alarmhandler-Code 56 vorhanden.

Im ROM der Behandlungsvorrichtung 50 sind damit alle für den Ablauf des Verfahrens verwendeten Daten gespeichert. Die maschinenlesbaren Inhalte der Ablaufdefinitionsausgabedatei 20 sowie eine Vorschrift, wie diese Daten zu behandeln sind, liegen ebenfalls im ROM vor. Ergänzt werden diese fixierten Daten durch dynamisch generierte, veränderliche Daten aus Abläufen und Überwachungen, wie Stellwerte, Sensorwerte, Umgebungsparameter und dergleichen. Diese veränderlichen Größen wirken auf die Ablaufermittlungseinrichtung 56.

Die Behandlungsvorrichtung 50 der Fig. 1 weist neben den oben genannten und weiteren, hier nicht weiter diskutierten Komponenten u. a. ein erstes Operationssystem 60 für einen Behandlungsabschnitt 59 der Behandlungsvorrichtung 50, auf welcher das Verfahren zur Behandlung abläuft, und ein zweites Operationssystem 62 für den Monitor 58, auf. Sie weist ferner ein erstes Schutzsystem 64 für den Behandlungsabschnitt 59 der Behandlungsvorrichtung 50 und ein zweites Schutzsystem 66 für den Monitor 58 auf. Eine erste Einrichtung 68 zum Speichern von Verfahrens- oder Betriebsabläufen der Behandlungsvorrichtung 50 und eine zweite derartige Einrichtung 70 für den Monitor 58 liegen jeweils zweifach vor. Dabei ist die Behandlungsvorrichtung 50 aus Gründen der Sicherheit in ihren wesentlichen Komponenten doubliert. Sie weist im Beispiel der Fig. 1 vier CPUs auf, von welchen zwei zum Betrieb des medizinischen Behandlungsverfahrens (eine CPU für das Operationssystem und eine weitere für das Schutzsystem) und separat dazu zwei weitere CPUs für den Anzeigebereich (Monitor 58), der keine Rückwirkung auf das Behandlungsverfahren hat, vorgesehen sind.

Das Beispiel der Fig. 1 ist auf ein Festlegen von Alarmzuständen gerichtet. Die vorliegende Erfindung ist jedoch hierauf nicht beschränkt, sondern kann auf beliebige Abläufe und Verfahren der medizinischen Behandlungsvorrichtung gerichtet sein.

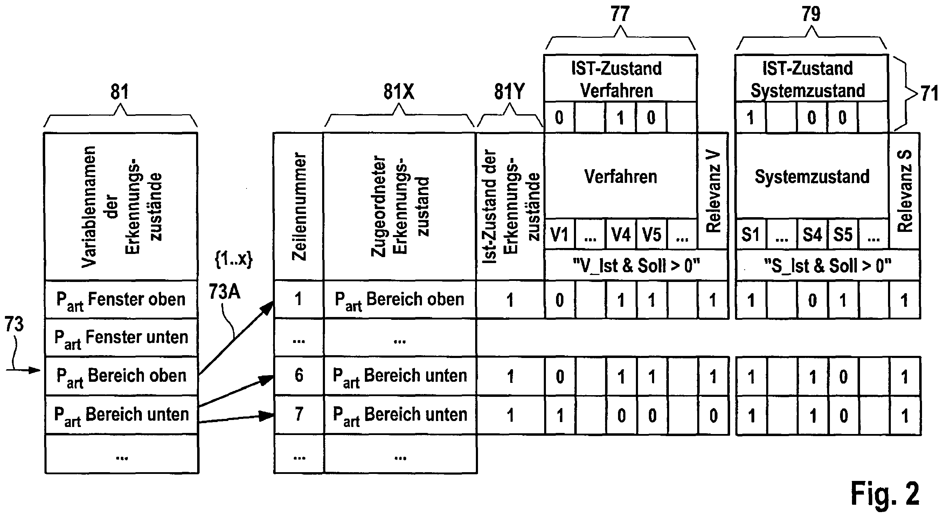

Fig. 2 zeigt am Beispiel einer permanenten Sensorüberwachung eine Relevanzbewertung, um bei einem positiven Ergebnis eine entsprechend vordefinierte Reaktion der medizinischen Behandlungsvorrichtung auszulösen. Wird ein oberer Grenzwert des mit dem Sensor gemessenen Wertes erreicht (beispielsweise der obere Grenzwert des arteriellen Druckfensters), so bedeutet dies noch keinen alarmpflichtigen Zustand, sondern stellt lediglich einen Erkennungszustand dar. Nun wird entschieden, ob dieser erkannte Zustand von Relevanz und somit ein Alarm auszulösen ist. Die Ablaufermittlungseinrichtung 56 (welche im Beispiel der Fig. 2 eine Alarmzustandsermittlungseinrichtung bzw. ein Alarmhandler-Code ist und im Folgenden auch so bezeichnet wird) liest permanent zeilenweise den Inhalt der in Fig. 2 dargestellten Alarmdefinitionstabelle aus und erhält in der Spalte "zugeordneter Erkennungszustand" 81X den Verweis auf einen definierten Erkennungszustand 81, welcher beispielsweise wie mit 73 bzw. 73A bezeichnet ein Überschreiten des oberen Grenzwertbereiches für den arteriellen Druck darstellt. Die Ist-Zustände 81Y (aktuelle Gerätezustände) der zugeordneten Erkennungszustände definieren die Relevanz der Zeilen. Eine gesetzte "1" enthält die Aufforderung, die Zeile zu bearbeiten. Vereinfacht dargestellt durchläuft die zeilenweise Abarbeitung drei Blöcke, nämlich den Verfahrensblock 77, einen Systemzustandsblock 79 und einen - in Fig. 2 nicht dargestellten - Reaktionsblock.

Dabei sind in der in Fig. 2 gezeigten Darstellung alle Erkennungszustände in einem ersten Block dargestellt, welche in Fig. 2 vereinfacht durch eine erste Spalte 81 abgebildet ist. Somit ist in der ersten Spalte einer jeden Zeile der Fig. 2 ein Erkennungszustand wiedergegeben, welcher z. B. als "Blutpumpenstillstand", "Überschreitung des Grenzwertfensters des arteriellen Drucks", "Blutpumpe dreht nicht" oder "Überschreitung des Grenzwertbereichs des arteriellen Drucks" usw. in jeweils allgemein verständlichem Klartext bezeichnet ist.

Definierte Erkennungszustände 81 können in mehreren Zeilen 73A in der Spalte "zugeordneter Erkennungszustand" 81X eingetragen sein und für beliebige Verfahren und für beliebige Systemzustände relevant sein.

Der Verfahrensblock 77 umfasst mehrere Spalten und gibt in den Spalten V1, ..., Vx alle denkbaren Verfahren oder Teilverfahren der medizinischen Behandlungseinrichtung an. Daher wird jedem bekannten oder möglichen medizinischen Behandlungsverfahren eine Spalte des Verfahrensblocks 77 zugeordnet. In dessen Zellen erfolgt ein Eintrag von "1" oder "0", je nachdem, ob der Erkennungszustand für das jeweilige Verfahren einer Spalte relevant ist ("1") oder nicht ("0"). Ist der Alarmhandler-Code in eine Zeile eingestiegen, so erkennt er für das Verfahren V1, das beispielsweise für "Single-Needle-Verfahren" oder "HD-Verfahren" steht, eine "0" und in den Verfahren V4 und V5 jeweils eine "1". Demnach wäre in zwei Verfahren der Erkennungszustand relevant. Jedoch benötigt der Alarmhandler-Code die Information, in welchem Verfahren sich die medizinische Behandlungsvorrichtung gerade befindet. Diese Information erhält der Alarmhandler-Code über dynamische Daten aus der Ablaufüberwachung. Diese bilden den IST-Zustand der Behandlungsvorrichtung ab. Die Ergebnisse sind in der Tabelle der Fig. 2 in der obersten Zeile, der Zeile 71, als "0" oder "1" eingetragen. Es gibt im Beispiel der Fig. 2 daher eine Übereinstimmung bei V4 und keine Übereinstimmung bei V5. Da aber bereits eine einzige Übereinstimmung relevant ist, wird der Alarmhandler-Code die Zeile nach rechts weiter abarbeiten.

Der Alarmhandler-Code oder Alarmhandler gelangt nun zum Systemzustandsblock 79. Dort sind alle möglichen Systemzustände bzw. alle möglichen oder relevanten zeitlichen Phasen der medizinischen Behandlungsverfahren wie "Befüllen", "Behandeln", "Abrüsten" usw. in Spalten festgehalten. Genau wie zuvor im Verfahrensblock 77 werden die Zustände bzw. deren Kombinationsmöglichkeiten mit "0" und "1" gekennzeichnet. Im Beispiel der Fig. 2 könnten also die Systemzustände S1 und S5 relevant sein, wobei angesichts der dynamischen Daten des IST-Systemzustands nur mit S1 eine Übereinstimmung und somit ein relevanter Zustand vorliegt. Das Ergebnis der Relevanzbewertung aus dem Verfahrensblock 77 und dem Systemzustandsblock 79 ist jeweils eine "1". Somit liegt eine Gesamtrelevanz vor. Systematisch würde die Tabelle jetzt nach rechts den identischen Aufbau besitzen, wobei dort dann alle weiteren Systemreaktionen eingetragen wären. Dies ist allein zur Vereinfachung der Darstellung in Fig. 2 nicht gezeigt.

Wird beim zeilenweisen Abarbeiten an einer Stelle einer Zeile festgestellt, dass keine Relevanz vorliegt, so wird der Rest dieser Zeile übersprungen und die nächste Zeile aus der Tabelle oder dem Verteilerschlüssel wird in gleicher Weise bearbeitet.

Die Aufgabe des Alarmhandler-Codes, welcher als Software im ROM der erfindungsgemäßen medizinischen Behandlungsvorrichtung abgelegt sein kann, beschränkt sich somit auf eine sehr einfache, standardisierte Abarbeitung von Bool'sehen Ausdrücken der Alarmdefinitionstabelle. Hierdurch ist der Alarmhandler-Code vorteilhaft leicht zu qualifizieren und auch in gleicher Weise an vielen anderen Stellen einsetzbar.

Es ist bei Verwendung der Tabellenform für die Ablaufdefinitionsausgabedatei ohne weiteres möglich, zusätzliche Zustände oder Reaktionen der medizinischen Behandlungsvorrichtung zu definieren. Dies erfordert lediglich die Aufnahme einer weiteren Spalte und/oder Zeile in den jeweiligen Blöcken. Eine solche Aufnahme kann in automatisierter Weise durch die Eingabeeinrichtung erfolgen.

Die Tabelle für die Ablaufdefinitionsausgabedatei kann ferner auch Zellen aufweisen, die erst während des Betriebs der entsprechenden medizinischen Behandlungsvorrichtung basierend auf der Ablaufüberwachung dynamisch belegt werden und welche den Ist-Zustand der medizinischen Behandlungsvorrichtung bei deren Betrieb abbilden.

Die Zusammenfassung aller Informationen in einer Definitionstabelle (ADT = Alarm Definition Table) hat u.a. folgende Vorteile:
- Alle für das Systemverhalten relevanten Informationen liegen zentral vor;
- Die Informationen liegen in einer Form vor, die auch für Nicht-Softwareentwickler lesbar ist (kein spezielles Know-How in einer bestimmten Programmiersprache erforderlich);
- Zur Realisierung des Systemverhaltens wird nur ein CodeTeil für die Gerätesoftware erstellt (auch Alarmhandler genannt), der die Informationen durchläuft, bewertet und die entsprechenden Reaktionen auslöst;
- Die Umsetzung der Tabelleninformationen in eine Form, die der Alarmhandler verarbeiten kann, erfolgt mit einem Konvertierungsprogramm bzw. einer Konvertierungseinrichtung (ACG = Advanced Code Generator); durch die automatisierte Umsetzung werden Fehler minimiert;
- Änderungen in der Spezifikation des Systemverhaltens erfolgen ausschließlich durch Modifikation der Tabelleninformation (mögliche Ausnahme hierbei: neue "Features" werden in die Tabelle eingebaut; dann ist auch eine Modifikation des Alarmhandlers erforderlich);
- Erweiterungen/Modifikationen können auf einfache Weise ohne Beeinträchtigung des Ablauf der übrigen Gerätesoftware durchgeführt werden; und
- Die Tabelle stellt gleichzeitig eine Dokumentation dar; es ist nicht mehr erforderlich, letztere separat zu erstellen oder zu pflegen.

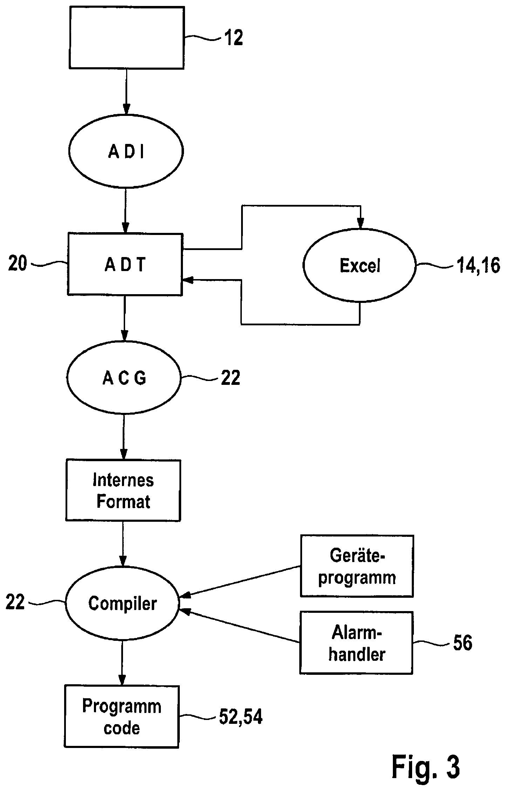

Fig. 3 zeigt in Form eines Schaubildes Daten- und Programmstrukturen in ihrem Bearbeitungsablauf am Beispiel der Festlegung von Alarmzuständen in einem Verfahren. Rechteckige Bereiche stehen in Fig. 3 für Datenobjekte, ovale Bereiche für Programme und Programmteile. Die Alarmdefinitionstabelle ADT 20 (im CSV-Format) wird beispielsweise mit Microsoft-Excel™ (oder einem vergleichbaren Tabellenkalkulations-Programm) oder einem einfachen Texteditor oder dergleichen als Eingabe- sowie Ausgabeeinrichtung (Bezugszeichen 14 bzw. 16) bearbeitet. Sie wird danach mit dem Hilfsprogramm ACG in eine Form "Internes Format" gewandelt, das für den verwendeten Compiler bzw. Konvertierungscode verwertbar ist. Letzterer erstellt aus den Daten der ADT 20 zusammen mit dem Codeteil für den Alarmhandler 56 und dem restlichen Geräteprogramm den eigentlichen, maschinenlesbaren Programmcode.

Die Informationen der Ablaufdefinitionsausgabedatei sind in drei Tabellen "Vorverarbeitung", "Erkennung", "Relevanz" gespeichert.

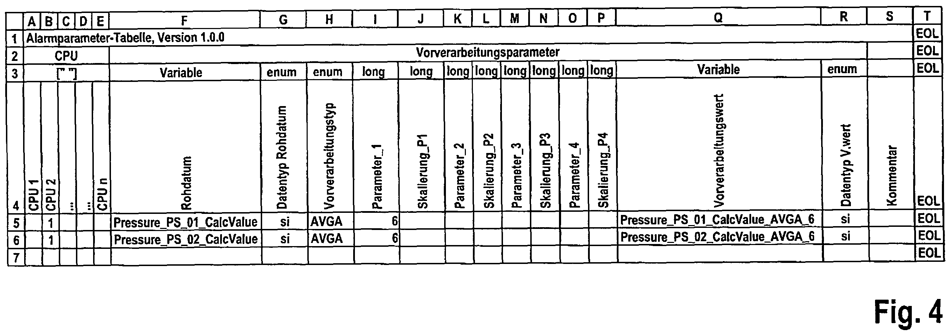

In der Vorverarbeitung, wie sie in Fig. 4 dargestellt ist, wird festgelegt, ob und ggf. welche "Roh-Messwerte" welche mathematische Aufbereitung wie Mittelung, Skalierung, Filterung oder dergleichen durchlaufen. Als weitere Filterarten sind z. B. Mittelungen, Polynomberechungen, etc. denkbar.

Im ersten Spaltenblock ("CPU") werden diejenigen Mikroprozessoren markiert, auf denen der Vorverarbeitungsprozess ablaufen soll. Im zweiten Spaltenblock ("Vorverarbeitungsparameter") wird festgelegt, welches Rohdatum (Variable) welchen Typs wie mathematisch aufbereitet wird. In obigem Beispiel wird ein Rohdruck "Pressure_PS_01_CalcValue" (Sensorwert eines Drucksensors PS1) durch einen Mittelwertfilter der Dimension 6 geschickt. Als Ausgabewert wird eine Variable "Pressure_PS_01_CalcValue_AVGA_6" definiert, die bereits in ihrer Namensgebung Informationen zum angewandten Filter enthält. Die Ausgangsvariable wird mittels Formelhinterlegung von der Toolkette automatisch erzeugt, sie kann aber auch manuell überschrieben werden. Die automatische Generierung von Variablennamen hat den Vorteil, dass doppelte Einträge identische Variablennamen erzeugen und spätestens beim abschließenden Konvertierungs- oder Kompilier-Vorgang zu einer Fehlermeldung führen.

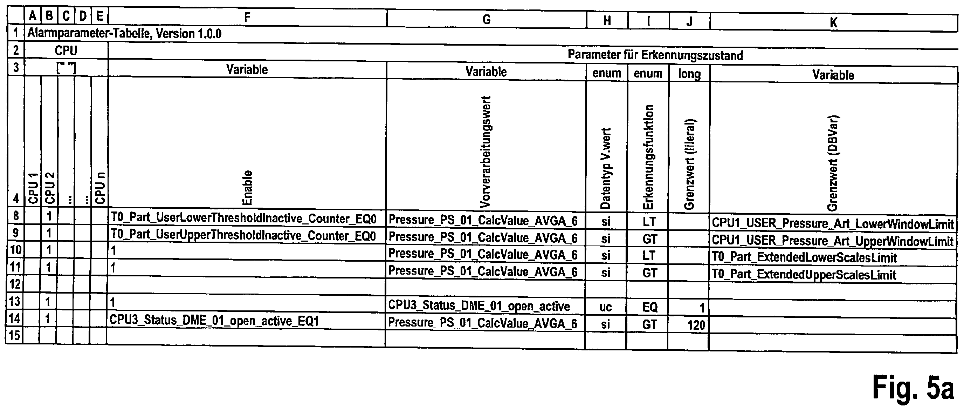

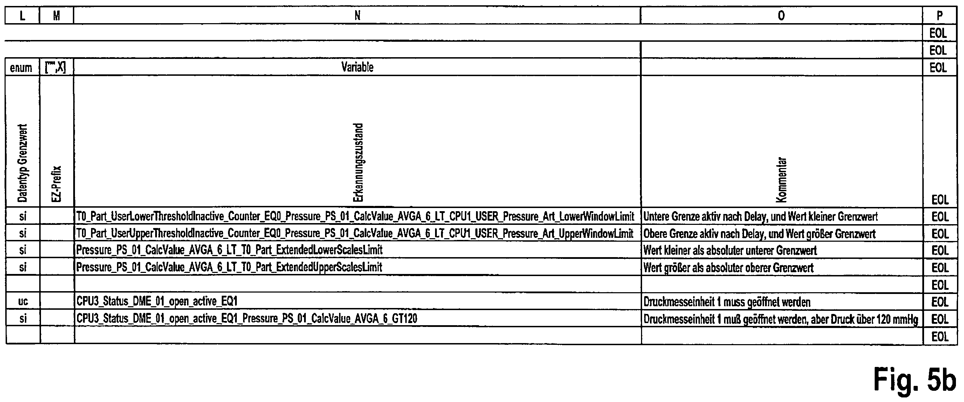

In der Tabelle "Erkennung", wie in Fig. 5a und 5b dargestellt, werden Werte zu Bedingungen verknüpft mit dem Ziel, einen Wahrheitswert (ja/nein; wahr/falsch) als Ergebnis zu erhalten. Diese resultierenden Wahrheitswerte nennen sich Erkennungszustände. Sie beschreiben den aktuellen Zustand eines Gerätes oder einer Komponente, z. B. "die Klemme A ist offen" oder "der Druck B ist höher als ein Grenzwert".

Die Erkennungstabelle (wie auch die Vorverarbeitungstabelle) wird zyklisch komplett durchlaufen, d. h. nach jedem Durchlauf stehen in den Erkennungszuständen aktuelle Werte zur Verfügung.

Pro Zeile kann eine Vergleichsoperation erfolgen. Typische Operationen sind "EQ" (gleich), "LT" (kleiner als), "GT" (größer als), aber auch "DGT" (Differenz größer als). Komplexere Bedingungen werden z. B. dadurch realisiert, dass mehrere Tabellenzeilen in Reihe geschaltet werden. Dies ergibt dann eine logische "UND"-Verknüpfung. "ODER"-Verknüpfungen werden durch separate Tabellenzeilen realisiert.

Dies soll zunächst allgemein erläutert werden. Ein konkretes Beispiel wird weiter unten erläutert.

In der Tabelle der Fig. 5a beginnt wiederum jede Zeile mit der Angabe der Ziel-CPU.

Danach folgt die Enable-Spalte, die zur UND-Verknüpfung verwendet wird. Steht hier in einer Zeile eine "1", so ist dies eine Zeile, in der eine Bedingungsdefinition beginnt.

Im Anschluss erfolgt die Spezifikation der Bedingung, bestehend aus einem Variablennamen, einer Vergleichsoperation und einem zweiten Wert. Dieser zweite Wert kann entweder wieder eine Variable oder eine Konstante (literal) sein.

Das Ergebnis der formulierten Bedingung wird dann in dem angegebenen Erkennungszustand (realisiert als Variable) abgelegt. Die Benennung der Erkennungszustands-Variablen erfolgt wie bei der Vorverarbeitung automatisch und generiert dabei eindeutige Bezeichnungen.

In einem konkreten Beispiel kann sich dies wie folgt darstellen, wobei das Beispiel ein Auszug aus einer Drucküberwachung ist. In der zweiten Zeile wird der Wert der Variablen "T0_Part_UserLowerThresholdInactive_ Counter" (dient zur Deaktivierung des Druckfensters für eine bestimmte Zeit) auf Gleichheit mit 0 geprüft. Das Ergebnis wird im Erkennungszustand "T0_Part_UserLowerThresholdInactive_Counter_EQ0" abgelegt. Der Erkennungszustand wird den Wert "Ja" annehmen, wenn der Zeitzähler für die Deaktivierung des arteriellen Druckes auf 0 heruntergezählt hat. In Zeile 8 des obigen Beispiels wird dieser Erkennungszustand als Enable verwendet, also zur Generierung einer UND-Verknüpfung. Im nun folgenden zweiten Teil der UND-Verknüpfung wird der aus der Vorverarbeitung bekannte gemittelte Druck "Pressure_PS_01_CalcValue_AVGA_6" daraufhin untersucht, ob er kleiner (LT) ist als der Wert der Variablen "CPUl_USER_Pressure_Art_LowerWindowLimit" (also dem vom Bediener vorgegebenen Wert). Das Wahrheitsergebnis der Gesamtbedingung wird dann im Erkennungszustand "T0_Part_UserLowerThresholdInactive_Counter_EQ0_Pressure_PS_0 1_CalcValue_AVGA_6_LT_CPU1_USER_Pressure_Art_LowerWindowLimit " abgelegt.

Auf diese Weise wird die in Prosa formulierte Bedingung "wenn die Totzeit abgelaufen ist, und sich der arterielle Druck unter dem vom Benutzer eingestellten unteren Grenzwert befindet" in der Erkennungstabelle dargestellt. Dazu sind ersichtlich keine besonderen Kenntnisse in der Programmierung erforderlich.

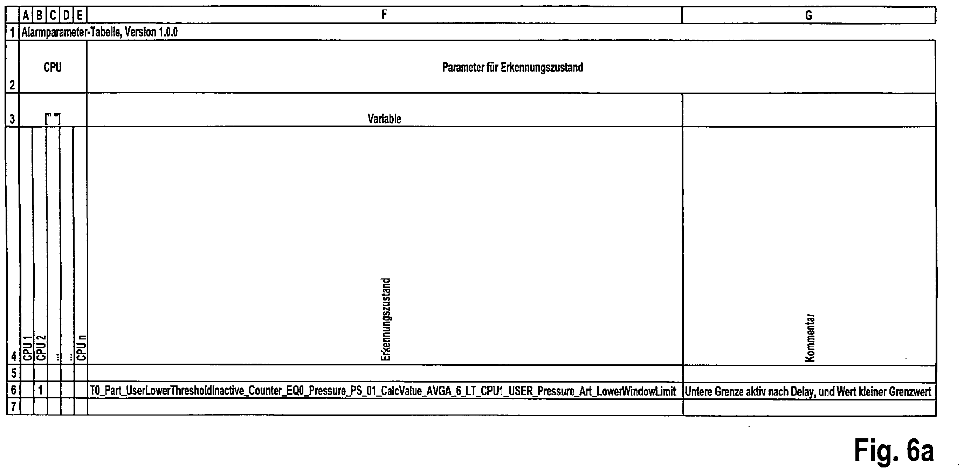

Auf die gleiche Weise kann die in den Zeilen 13 und 14 aufgestellte Bedingung gedeutet werden als "wenn Druckmesseinheit 1 aufgefahren werden soll, der anstehende Druck aber größer als 120 [mmHg] beträgt, dann ..." <muss das verhindert werden, da sonst das Schlauchsystem beschädigt wird>. Diese hier informativ hinzugefügte Konsequenz ist nicht Bestandteil der Erkennungstabelle, sondern in der folgenden Relevanztabelle der Fig. 6a bis 6f abgebildet.

In den bisherigen beiden Stufen sind Rohdaten vorverarbeitet und Gerätezustände anhand (komplexer) Bedingungen ermittelt. In dieser Stufe "Relevanz" (siehe Fig. 6a bis 6f) erfolgt nun die Bewertung dieser Zustände und die Formulierung der entsprechenden "dann"-Aktion.

Nach dem grundlegenden Gedanken prüft der Alarmhandler bei der Abarbeitung zur Laufzeit der Gerätesoftware den Inhalt der Relevanztabelle zeilenweise. Dabei wird die Prüfung nur soweit durchgeführt, wie die definierten Bedingungen gültig sind. Ist z. B. das Behandlungsverfahren HD-Verfahren aktiv, in der bearbeiteten Zeile in der Spalte HD-Verfahren aber keine Markierung gesetzt, so wird die Bearbeitung der Zeile an dieser Stelle abgebrochen und mit der nächsten Zeile fortgefahren.

Bei vollständiger (und positiver) Abarbeitung aller Bedingungen in einer Zeile gelangt der Alarmhandler im Weiteren auf Einträge, die zu einer Systemreaktion führen. Hier ist festgelegt, welche Aktionen aufgrund der definierten Bedingungen auszuführen sind.

Der Aufbau der Relevanztabelle der Fig. 6a bis 6f wird im Folgenden an einem Beispiel erläutert.

In Fig. 6a wird wiederum zunächst die CPU festgelegt, bei der die Systemreaktion ausgelöst werden soll. Danach wird der Erkennungszustand definiert, der sich aus der Erkennungstabelle der Fig. 5 ergibt.

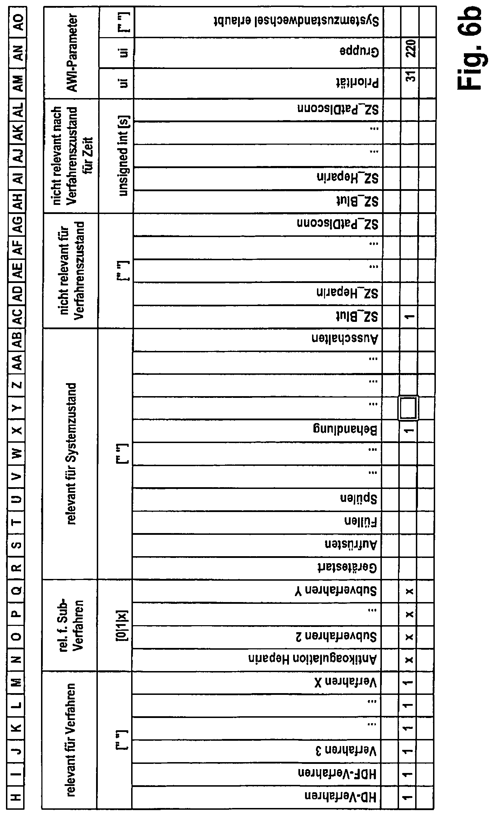

In den folgenden drei Spaltenblöcken der Fig. 6b wird definiert, ob dieser Erkennungszustand für das aktuelle Behandlungsverfahren, für das aktuelle Subverfahren oder im aktuellen Systemzustand betrachtet werden muss (d. h. relevant ist).

Die dort aufgeführten, exemplarischen Verfahren reichen von "HD-Verfahren" über "HDF-Verfahren" bis "Verfahren X"; der tatsächliche Verfahrensumfang ist erfindungsgemäß aber veränderbar und kann Verfahren wie "Coraffin", "SCUF" und dergleichen mehr umfassen. Ist in einer Spalte eine "1" eingetragen, und stimmt das aktuell ausgewählte Behandlungsverfahren mit dem in dieser Spalte genannten überein, so wird der in dieser Zeile definierte Erkennungszustand vom Alarmhandler weiter betrachtet. Ist die entsprechende Zelle nicht markiert, so kann der Alarmhandler die Auswertung dieser Zeile abbrechen, da sie für das aktuelle Verfahren nicht relevant ist.

Für das Subverfahren (beispielsweise Antikoagulation Heparin, CiCa und FPSA) gilt eine erweiterte Definition: wird in der entsprechenden Spalte eine "1" eingetragen, so ist diese Zeile beim aktiven Subverfahren relevant. Wird dagegen ein "x" eingetragen, so ist diese Zeile unabhängig davon relevant, ob das entsprechende Subverfahren aktiv ist oder nicht. Kein Eintrag (oder "0")heißt, dass diese Zeile für das angegebene Subverfahren nicht relevant ist.

Analog erfolgt die Bewertung des Programmzustands (auch Systemzustand genannt). In der Tabelle der Fig. 6b existiert für jeden definierten Programmzustand eine Spalte. Dies sind stellvertretend die Spalten "Gerätestart" bis "Ausschalten". Für das erfindungsgemäße tabellengestützte Alarmhandling sind wiederum weder die Bedeutung noch die Anzahl der Programmzustände wichtig. Wichtig ist jedoch, dass es eine Unterscheidung gibt, in welchem Systemzustand sich die medizinische Behandlungsvorrichtung aktuell befindet. Eine "1" in der entsprechenden Zeilen/Spalten-Kombination bedeutet, dass die definierte Zeile dann relevant ist, wenn der aktuelle Programmzustand mit dem in der Spalte genannten übereinstimmt.

Dies zeigt wiederum einen der Vorteile einer tabellengestützten Definition. Soll eine Überwachung (definiert in der Erkennungstabelle und resultierend in einem Erkennungszustand) z. B. für ein weiteres Verfahren aktiviert werden, so genügt ein einfacher Eintrag in der Matrix.

Gleiches gilt für den Programmzustand (wenn z. B. die Druckerkennung nicht nur in "Behandlung", sondern auch in "Spülen" aktiv sein soll. Auch eine Rückwirkungsfreiheit zu anderen Programmteilen der Gerätesoftware kann sichergestellt werden, da nur zusätzliche "Freischaltungen" erfolgen, die bestehenden dadurch aber nicht verändert werden.

Die nächsten beiden Spaltenblöcke der Fig. 6b stellen eine Ausnahmeregelung dar. In der Spalte "nicht relevant für Verfahrenszustand" kann definiert werden, ob eine Reaktion unterbleiben soll. Befindet sich das System bereits in einem bestimmten, sicheren Zustand (wie beispielsweise SZ_Blut), so kann beispielsweise ein Druckproblem unbeachtet bleiben, da der sichere Zustand bereits hergestellt ist. Diese Spalten können dazu dienen, mehrfache Systemreaktionen und missdeutige Folgemeldungen zu verhindern. Im folgenden Spaltenblock "nicht relevant nach Verfahrenszustand für Zeit" wird festgelegt, dass z. B. nach einem Verfahrenszustand "SZ_Blut" bestimmte Erkennungszustände für eine gewisse Zeit nicht betrachtet werden sollen, z. B. weil sich das System in dieser Zeitspanne zunächst stabilisieren muss. Im Wesentlichen werden diese Spalten also dafür verwendet, die Überwachung während des erneuten Anlaufens eines angehaltenen Verfahrens vorübergehend etwas "toleranter" zu gestalten.

Der letzte Block in Fig. 6b umfasst die "AWI-Parameter"; die "Priorität" benennt hierbei die Wichtigkeit einer mit dieser Zeile verknüpften Meldung. Die mit dieser Zeile verknüpften Systemreaktionen sind nicht priorisiert und werden in jedem Fall ausgeführt.

Des Weiteren können die Zeilen der Relevanztabelle in Gruppen eingeteilt werden. Dadurch können über Meldungen, die vom Anwender bestätigt werden, automatisch alle weiteren Tabellenzeilen, die aktiv und derselben Gruppe zugeordnet sind und eine gleiche oder niedrigere Priorität haben, mitbestätigt werden. Allgemein werden diese Informationen also zur Behandlung von Folgealarmen verwendet.

Die letzte Spalte "erlaubt Systemzustandswechsel" definiert, ob das Gerät bei einer als relevant erkannten Zeile den Systemzustand wechseln darf. An einigen Stellen ist es sinnvoll, dieses Geräteverhalten zu blockieren. Dies kann beispielsweise dann der Fall sein, wenn in einer Tabellenzeile ein Erkennungszustand definiert ist, nach welchem der Filter nicht vollständig befüllt wurde. Die Vorrichtung kann das Füllen nicht beenden, solange diese Bedingung gegeben ist.

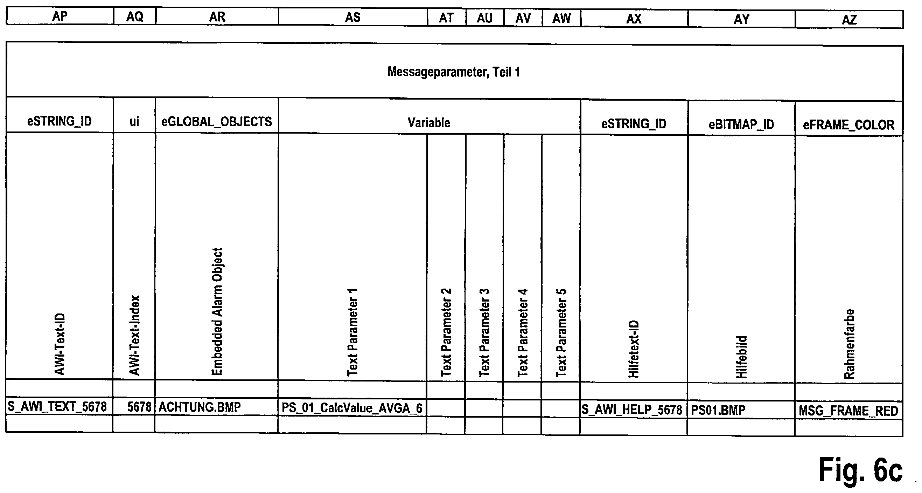

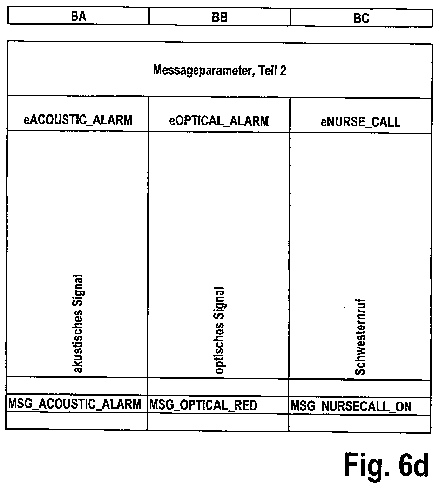

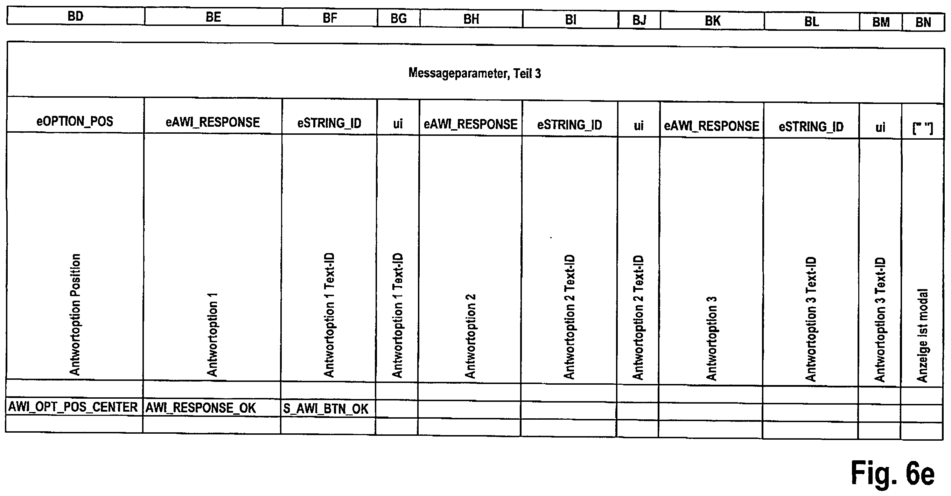

Die Spalten in den Fig. 6c, 6d und 6e stellen eine detaillierte Spezifikation der Anzeigereaktion bzw. der Schnittstelle zum Anwender dar. Darunter fallen Meldungstexte, Farben und Formate auf dem Bildschirm, Ampelsignalisierung, akustische Reaktion, Schwesternruf, etc.

In den Fig. 6d und 6e sind Meldungsdarstellungen für den Bildschirm wiedergegeben. Dabei gilt:

| | |
|---|---|
| ∘ AWI-Text-ID | ist eine eindeutige Nummer, die den Meldungstext definiert; der eigentliche Text wird anhand dieser Nummer aus einer Liste entnommen; |
| ∘ AWI-Text-Index | kann ein Duplikat der AWI-Text-ID sein; |
| ∘ Embedded Alarm Object | im Meldungsfenster wird neben dem Text ein zusätzliches Objekt dargestellt; Beispiel: bei einem Druckalarm wird in das Meldungsfenster ein "Achtung"-Symbol eingebettet, das zusätzlich auf die Wichtigkeit des Alarms hinweisen soll; |
| ∘ Text-Parameter 1-5 | Meldungstexte können Formatierungen enthalten, um aktuelle Werte in den Meldungstext einzubetten; Beispiel: "Druck überschreitet <p1> mmHg". An dieser Stelle wird der Wert des Text-Parameters 1 angezeigt. Es stehen derzeit maximal 5 Parameter zur Verfügung. Eingetragen werden die Variablen-Namen; |
| ∘ Hilfetext-ID | ist eine eindeutige Nummer, die den Hilfetext definiert; der eigentliche Text wird anhand dieser Nummer aus einer Liste entnommen; kann auch nicht definiert sein, dann existiert zu dieser Meldung keine Hilfe; |
| ∘ Hilfe-Bild | im Hilfefenster kann neben dem Text optional eine Grafik dargestellt werden, die hier spezifiziert wird; |
| ∘ Rahmenfarbe | Das Meldungsfenster wird mit der angegebenen Farbe gezeichnet. Es besteht die Auswahlmöglichkeiten grau, gelb, rot. Erweiterungen um zusätzliche Farben und/oder Rahmen-Eigenschaften sind erfindungsgemäß möglich; |
| ∘ Akustisches Signal | legt fest, welches Tonsignal zu dieser Meldung erzeugt wird; |
| ∘ Optisches Signal | legt fest, welche Farbe/Farbkombination die Ampel bei dieser Meldung anzeigen soll; |
| ∘ Schwesternruf | legt fest, ob bei dieser Meldung der Schwesternruf aktiviert werden muss; |
| ∘ Antwortoption-Position | legt fest, in welcher Anordnung die Bestätigungstasten im Meldungsfenster erscheinen; hier gibt es Auswahlmöglichkeiten wie zentriert, linksbündig, rechtsbündig, Abstände, usw. |
| ∘ Antwortoption 1 | Signal, das bei Betätigung der ersten Betätigungstaste im Geräte ausgelöst wird; |
| ∘ Antwortoption 1 Text-ID | Beschriftung der ersten Bestätigungstaste; |
| ∘ Antwortoption 2 | s.o. |
| ∘ Antwortoption 2 Text-ID | s.o. |
| ∘ Antwortoption 3 | s.o. |
| ∘ Antwortoption 3 Text-ID | s.o. |
| ∘ Anzeige ist modal | Anzeigeoption; Anzeige kann durch andere Objekte auf dem Bildschirm überdeckt werden; |

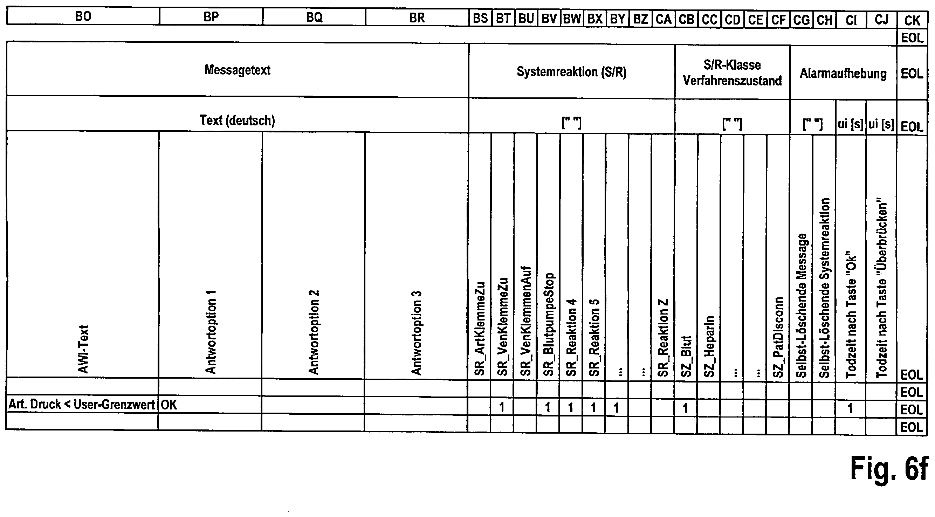

Der nächste Spaltenblock der Fig. 6f mit der Überschrift "Messagetext" beginnt zunächst mit vier Spalten, die von der Anmelderin nur zu Entwicklungszwecken verwendet werden und praktisch die "Mini-Kopie" der Meldungsdefinition enthalten. Der nächste Spaltenblock beinhaltet die Systemreaktion. Folgende komponentenbasierte Systemreaktionen sind exemplarisch definiert:

| | |
|---|---|
| ∘ SR_ArtKlemmeZu | Schließen der arteriellen Klemme; |
| ∘ SR_VenKlemmeZu | Schließen der venösen Klemme; |
| ∘ SR_VenKlemmeAuf | Öffnen der venösen Klemme (z. B. zum Druckabbau); |
| ∘ SR_BlutpumpeStop | Anhalten der Blutpumpe. |

Weitere komponentenbasierte Systemreaktionen wie die folgenden sowie wiederum weitere können natürlich ebenfalls definiert sein:

| | |
|---|---|
| ∘ SR_DialysatpumpeStop | Anhalten der Dialysatpumpe; |
| ∘ SR_SubstituatpumpeStop | Anhalten der Substituatpumpe; |
| ∘ SR_FiltratpumpeStop | Anhalten der Filtratpumpe; |
| ∘ SR_FPSAKlemmeZu | Schließen der FPSA-Klemme; |
| ∘ SR_FPSAKlemmeAuf | Öffnen der FPSA-Klemme; |
| ∘ SR_FPSAPumpeStop | Anhalten der FPSA-Pumpe; |
| ∘ SR_HeizungAus | Ausschalten der Heizung(en); |
| ∘ SR_CiPumpeStop | Anhalten der Citratpumpe; |
| ∘ SR_CaPumpeStop | Anhalten der Calciumpumpe; |
| ∘ SR_HepPumpeStop | Anhalten der Heparinpumpe; |
| ∘ SR_24V_Off | Abschalten der 24V-Versorgung (Not-Aus) ; |
| ∘ SR_PowerOff | Komplettabschaltung (ein weiterer Not-Aus); und |
| ∘ SR_PGM_Error | Übergang in den Fehlerzustand; keine Fortführung der Behandlung möglich. |

Für die Umsetzung des Alarmhandlings ist es dabei nicht relevant, welche speziellen Systemreaktionen definiert sind. Dies erfolgt in Abhängigkeit von der Geräte-Komponentenausstattung bzw. der Art des Behandlungsverfahrens. Wichtig ist vielmehr, dass alle Aktoren, die in irgendeiner Weise das Behandlungsverfahren beeinflussen können, aufgelistet und bei der Systemreaktion berücksichtigt werden.

Bei der oben stehenden Diskussion der Fig. 6b wurde bereits auf die Ausnahmeregelung bezüglich des Verfahrenszustands hingewiesen. Im nächsten Spaltenblock "S/R-Klasse" wird genau dieser Verfahrenszustand definiert, d. h. zu den komponentenbasierten, einzelnen Systemreaktionen wird der Verfahrenszustand gesetzt, der dann für die weitere Relevanzbewertung verwendet wird. Der letzte Spaltenblock beinhaltet Optionen, die zur Löschung der generierten Meldung führen können.

Bei einer Selbst-Löschenden Meldung erlöscht die Meldung, wenn der Erkennungszustand auf "falsch" wechselt. Bei einer Selbst-Löschenden Systemreaktion wird die ausgelöste Systemreaktion zurückgenommen, wenn der Erkennungszustand auf "falsch" wechselt. Mittels der Totzeit nach "OK" kann spezifiziert werden, wie lange der Erkennungszustand nicht berücksichtigt wird, wenn die Meldung mit OK bestätigt wird.

Über die Totzeit nach "Überbrücken" kann spezifiziert werden, wie lange der Erkennungszustand deaktiviert ist, wenn die Meldung mit "Überbrücken" bestätigt wird.

## Patentansprüche

1. Medizinische Behandlungsvorrichtung (50) mit einer Speichereinrichtung, **gekennzeichnet durch** einen Datenträger mit wenigstens einer maschinenlesbaren Konfigurationsdatei zum Umprogrammieren von durch die Gerätesoftware beeinflussten Ablaufmustern der medizinischen Behandlungsvorrichtung (50), welche in einem Rechnersystem (10) für die medizinische Behandlungsvorrichtung erzeugt wurde, wobei das Rechnersystem (10) aufweist
eine Datenbank (12) zum Bereithalten wenigstens eines Parametersatzes für behandlungsspezifische Abläufe;
eine Eingabeeinrichtung (14) zum Eingeben von Daten das behandlungsspezifische Ablaufverhalten der medizinischen Behandlungsvorrichtung betreffend als Parametersatz;
eine Ausgabeeinrichtung (16), konfiguriert zum Erzeugen und Ausgeben einer Ablaufdefinitionsausgabedatei (20) unter Einbeziehung der eingegebenen Daten; und
eine Konvertierungseinrichtung (22), konfiguriert zum Erzeugen der wenigstens einen maschinenlesbaren Konfigurationsdatei in einem Speichermedium unter Verwenden wenigstens der eingegebenen Daten,
wobei die maschinenlesbare Konfigurationsdatei für den Betrieb der medizinischen Behandlungsvorrichtung (50) in der Speichereinrichtung der medizinischen Behandlungsvorrichtung (50) abgelegt oder gespeichert ist.

2. Medizinische Behandlungsvorrichtung (50) nach Anspruch 1, wobei die Ausgabeeinrichtung (16) vorbereitet ist, eine Ablaufdefinitionsausgabedatei (20) mit wenigstens einer Tabelle mit mehreren Zeilen und/oder Spalten auszugeben.

3. Medizinische Behandlungsvorrichtung (50) nach Anspruch 1 oder 2, wobei die Ablaufdefinitionsausgabedatei (20) eine Alarmdefinitionstabelle umfasst.

4. Medizinische Behandlungsvorrichtung (50) nach einem der Ansprüche 1 bis 3, wobei der Datenträger eine Mehrzahl von maschinenlesbaren Konfigurationsdateien aufweist, wobei sich wenigstens zwei Konfigurationsdateien aus der Mehrzahl von maschinenlesbaren Konfigurationsdateien voneinander unterscheiden.

5. Medizinische Behandlungsvorrichtung (50) nach einem der vorangegangenen Ansprüche, welche als Dialysevorrichtung ausgestaltet ist.

## Claims

1. A medical treatment apparatus (50) with a memory means, **characterized by** a data carrier having at least one machine-readable configuration file for reprogramming process patterns of the medical treatment apparatus (50) influenced by the equipment software, which has been generated in a computer system (10) for the medical treatment apparatus, wherein the computer system (10) comprises:
- a database (12) for providing at least one set of parameters for treatment-specific processes;
- an input device (14) for inputting data relating to the treatment-specific processes behavior of the medical treatment apparatus, considered as a set of parameters;
- an output device (16), configured to generate and output a process definition output file (20) taking the inputted data into account; and
- a conversion device (22), configured to generate the at least one machine-readable configuration file in a memory medium using at least the inputted data,
wherein the machine-readable configuration file for the operation of the medical treatment apparatus (50) is stored or memorized in the memory means of the medical treatment apparatus (50).

2. The medical treatment apparatus (50) according to claim 1, wherein the output device (16) is prepared to output a process definition output file (20) comprising at least one table having a plurality of rows and/or columns.

3. The medical treatment apparatus (50) according to claim 1 or 2, wherein the process definition output file (20) comprises an alarm definition table.

4. The medical treatment apparatus (50) according to anyone of claims 1 to 3, wherein the data carrier comprises a plurality of machine-readable configuration files, wherein at least two configuration files from the plurality of machine-readable configuration files differ from each other.

5. The medical treatment apparatus (50) according to anyone of the preceding claims, which is designed as a dialysis apparatus.

## Revendications

1. Un appareil de traitement médical (50) avec un dispositif de mémorisation, **caractérisé par** un support de données comportant au moins un fichier de configuration lisible par machine pour la reprogrammation de modèles de processus de l'appareil de traitement médical (50) influencés par le logiciel de l'équipement, qui a été généré dans un système informatique (10) pour l'appareil de traitement médical, où le système informatique (10) comprend:
- une base de données (12) pour mettre à disposition d'au moins un ensemble de paramètres pour des processus spécifiques au traitement;
- un dispositif d'entrée (14) pour entrer des données relatives au comportement du processus spécifique au traitement de l'appareil de traitement médical, comme ensemble de paramètres;
- un dispositif de sortie (16), configuré pour générer et délivrer un fichier de sortie de définition de processus (20) incluant les données entrées; et
- un dispositif de conversion (22), configuré pour générer au moins le fichier de configuration lisible par machine sur un support de mémorisation en utilisant au moins les données entrées,
où le fichier de configuration lisible par machine pour le fonctionnement de l'appareil de traitement médical (50) est stocké ou mémorisé dans le dispositif de mémorisation de l'appareil de traitement médical (50).

2. L'appareil de traitement médical (50) selon la première revendication, où le dispositif de sortie (16) est préparé pour délivrer un fichier de sortie de définition de processus (20) comportant au moins un tableau ayant une pluralité de lignes et/ou de colonnes.

3. L'appareil de traitement médical (50) selon la revendication 1 ou 2, où le fichier de sortie de définition de processus (20) comprend un tableau de définition d'alarme.

4. L'appareil de traitement médical (50) selon l'une quelconque des revendications 1 à 3, où le support de données comprend une pluralité de fichiers de configuration lisibles par machine, où au moins deux fichiers de configuration de la pluralité de fichiers de configuration lisibles par machine diffèrent les uns des autres.

5. L'appareil de traitement médical (50) selon l'une quelconque des revendications précédentes, qui est conçu comme un appareil de dialyse.
